(19) Europäisches Patentamt
European Patent Office
Office européen des brevets

(11) EP 3 123 920 A1

(12) EUROPEAN PATENT APPLICATION
published in accordance with Art. 153(4) EPC

(43) Date of publication:
01.02.2017 Bulletin 2017/05

(21) Application number: 15768959.7

(22) Date of filing: 03.02.2015

(51) Int Cl.:
*A61B 1/00* (2006.01) *G02B 23/24* (2006.01)

(86) International application number:
PCT/JP2015/052962

(87) International publication number:
WO 2015/146290 (01.10.2015 Gazette 2015/39)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 28.03.2014 JP 2014068942

(71) Applicant: Olympus Corporation
Hachioji-shi
Tokyo 192-8507 (JP)

(72) Inventors:
- SASAMOTO, Tsutomu
Tokyo 192-8507 (JP)
- ORIHARA, Tatsuya
Tokyo 192-8507 (JP)
- NAKAMURA, Minoru
Tokyo 192-8507 (JP)
- ISAKA, Toru
Tokyo 192-8507 (JP)

(74) Representative: Schicker, Silvia
Wuesthoff & Wuesthoff
Patentanwälte PartG mbB
Schweigerstraße 2
81541 München (DE)

# (54) IMAGING UNIT FOR ENDOSCOPES

(57) Manufacturing errors are suppressed, and the precision of positioning between an image-acquisition device and an objective lens is improved. An endoscopic image-acquisition unit includes an objective-lens-unit frame (2) that holds an objective lens; and an image-acquisition-device holding frame (3) that is fitted with the objective-lens-unit frame and that holds an image-acquisition device, wherein the objective-lens-unit frame and the image-acquisition-device holding frame are bonded and fixed with a thermosetting resin (12) that is applied to a fitting region of these frames, and wherein, of the outer surface of the frame that is located on the outer side when the objective-lens-unit frame and the image-acquisition-device holding frame are fitted together, the outer surface of the fitting region and the outer surface of the region other than the fitting region satisfy the following conditional expression:

$$\alpha/\beta > 2 \qquad (1)$$

where $\alpha$ signifies the infrared absorption rate per unit area of the outer surface of the fitting region, and $\beta$ signifies the infrared absorption rate per unit area of the outer surface of the region other than the fitting region.

FIG. 1

α REGION
β REGION
2
12
11
3
L3
L2
L1
L4
10
5
4

EP 3 123 920 A1

## Description

{Technical Field}

The present invention relates to image-acquisition units that are applied to endoscopes.

{Background Art}

Recently, it has become desirable to reduce the diameters of the distal ends of inserted portions of medical endoscopes, such as transnasal endoscopes, from the viewpoint of reducing the burden of patients, etc. Accordingly, small image-acquisition devices (CCDs and CMOSs) for endoscopes have been developed, and the pixel pitches thereof are decreasing year by year. In accordance with this decrease in pixel pitch, tolerable assembly errors for the distances between lenses, between an image-acquisition device and an objective lens, etc. are decreasing, and assembly errors on the order of a few micrometers to submicrometers are now becoming a problem.

As disclosed in Patent Literature 1, an endoscopic image-acquisition unit is configured such that an objective-lens-unit frame that holds an objective lens and an image-acquisition device are fitted and fixed together. More specifically, a thermosetting resin is applied to fitting regions of the objective-lens-unit frame and the image-acquisition-device holding frame, and with the optical axes of an objective optical system and an image-acquisition device oriented so as to achieve a desired focus, the frames are fixed together by using assembly jigs and are heated in a drying furnace, etc. Thus, the thermosetting resin is cured, whereby the objective-lens-unit frame and the image-acquisition-device holding frame are bonded to and fixed together.

{Citation List}

{Patent Literature}

{PTL 1}
Japanese Unexamined Patent Application, Publication No. Hei 9-192093

{Summary of Invention}

{Technical Problem}

With the conventional endoscopic image-acquisition unit described above, however, since the objective-lens-unit frame and the image-acquisition-device holding frame are heated in a drying furnace while being fixed together by using assembly jigs when curing the thermosetting resin, parts and the jigs experience thermal expansion. This thermal expansion may cause deviations of the objective-lens-unit frame and the image-acquisition-device holding frame from desired positions, which may result in manufacturing errors exceeding tolerances.

The present invention has been made in view of the situation described above, and an object thereof is to suppress manufacturing errors, thereby improving the precision of positioning between an image-acquisition device and an objective lens.

{Solution to Problem}

In order to achieve the above object, the present invention employs the following solutions.

According to a first aspect of the present invention, there is provided an endoscopic image-acquisition unit including an objective-lens-unit frame that holds an objective lens; and an image-acquisition-device holding frame that is fitted with the objective-lens-unit frame and that holds an image-acquisition device, wherein the objective-lens-unit frame and the image-acquisition-device holding frame are bonded and fixed with a thermosetting resin that is applied to a fitting region of these frames, and wherein, of the outer surface of the frame that is located on the outer side when the objective-lens-unit frame and the image-acquisition-device holding frame are fitted together, the outer surface of the fitting region and the outer surface of the region other than the fitting region satisfy the following conditional expression:

$$\alpha/\beta > 2 \qquad (1)$$

where $\alpha$ signifies the infrared absorption rate per unit area of the outer surface of the fitting region, and $\beta$ signifies the

infrared absorption rate per unit area of the outer surface of the region other than the fitting region.

According to the first aspect of the present invention, of the outer surface of the frame that is located on the outer side when the objective-lens-unit frame and the image-acquisition-device holding frame are fitted together, the outer surface of the fitting region and the outer surface of the region other than the fitting region are configured to satisfy conditional expression (1). Thus, the infrared absorption rate is higher in the fitting region of the objective-lens-unit frame and the image-acquisition-device holding frame compared with the region other than the fitting region. That is, in this configuration, the fitting region of the objective-lens-unit frame and the image-acquisition-device holding frame is heated more easily by infrared irradiation compared with the region other than the fitting region. Accordingly, by applying a thermosetting resin to the fitting region of the objective-lens-unit frame and the image-acquisition-device holding frame, positioning and fixing these frames by using jigs, and irradiating the fitting region with infrared rays, it is possible to efficiently heat only the fitting region.

As described above, when bonding and fixing the fitting region of the objective-lens-unit frame and the image-acquisition-device holding frame by curing the thermosetting resin, it suffices to irradiate the fitting region with infrared rays, and it is unnecessary to heat the entire objective-lens-unit frame and image-acquisition-device holding frame fixed together by using jigs. Furthermore, by making the infrared absorption rate of the fitting region greater than the infrared absorption rate of the region other than the fitting region, the fitting region is heated effectively, whereas heating of the region other than the fitting region is suppressed, so that transfer of heat to the jigs is minimized. Accordingly, it is possible to suppress positional deviations due to thermal expansion of jigs and parts, which serves to suppress manufacturing errors, so that the precision of positioning between the objective lens and the image-acquisition device can be improved.

In the above first aspect, conditional expression (2) below may be satisfied:

$$\alpha/\beta > 4 \qquad (2)$$

In this case, it is possible to make the infrared absorption rate of the fitting region greater compared with the region other than the fitting region. Accordingly, it is possible to suppress positional deviations due to thermal expansion of jigs and parts, which serves to suppress manufacturing errors, so that the precision of positioning between the objective lens and the image-acquisition device can be improved.

In the above first aspect, conditional expression (3) below may be satisfied:

$$Ra > 3 \times Rb \qquad (3)$$

where Ra signifies the maximum height of the surface roughness of the outer surface of the fitting region, and Rb signifies the maximum height of the surface roughness of the outer surface of the region other than the fitting region. The maximum height here refers to a value ($\mu$m) indicating the difference between a maximum value and a minimum value of the surface roughness.

This makes it possible to increase the surface area of the outer surface of the fitting region, so that it is possible to make the infrared absorption rate of the fitting region greater compared with the region other than the fitting region.

According to a second aspect of the present invention, there is provide an endoscopic image-acquisition unit including an objective-lens-unit frame that holds an objective lens; and an image-acquisition-device holding frame that is fitted with the objective-lens-unit frame and that holds an image-acquisition device, wherein the objective-lens-unit frame and the image-acquisition-device holding frame are bonded and fixed with a thermosetting resin that is applied to a fitting region of these frames, and wherein the outer surface of the frame that is located on the outer side when the objective-lens-unit frame and the image-acquisition-device holding frame are fitted together and the outer surface of the region other than the fitting region in the outer surface of the frame that is located on the inner side satisfy conditional expression (4) below:

$$\rho/\gamma > 1.5 \qquad (4)$$

where $\rho$ signifies the infrared absorption rate per unit area of the outer surface of the frame that is located on the outer side, and $\gamma$ signifies the infrared absorption rate per unit area of the outer surface of the region other than the fitting region in the outer surface of the frame that is located on the inner side.

According to the second aspect of the present invention, the outer surface of the frame that is located on the outer side when the objective-lens-unit frame and the image-acquisition-device holding frame are fitted together and the outer surface of the region other than the fitting region in the outer surface of the frame that is located on the inner side are configured to satisfy conditional expression (4), so that the infrared absorption rate is higher in the fitting region of the objective-lens-unit frame and the image-acquisition-device holding frame compared with the region other than the fitting region. That is, in this configuration, the fitting region of the objective-lens-unit frame and the image-acquisition-device holding frame is heated more easily by infrared irradiation compared with the region other than the fitting region. Accordingly, by applying a thermosetting resin to the fitting region of the objective-lens-unit frame and the image-acquisition-device holding frame, positioning and fixing these frames by using jigs, and irradiating the fitting region with infrared rays, it is possible to efficiently heat only the fitting region.

As described above, when curing the thermosetting resin, it suffices to irradiate the fitting region with infrared rays, and it is unnecessary to heat the entire objective-lens-unit frame and image-acquisition-device holding frame fixed together by using jigs. Furthermore, by increasing the infrared absorption rate of the fitting region such that the infrared absorption rate of the region other than the fitting region is lower compared with the fitting region, transfer of heat to the jigs is minimized. Accordingly, it is possible to suppress positional deviations due to thermal expansion of jigs and parts, which serves to suppress manufacturing errors, so that the precision of positioning between the objective lens and the image-acquisition device can be improved.

In the above second aspect, conditional expression (5) below may be satisfied:

$$\rho/\gamma > 2 \qquad (5)$$

In this case, it is possible to make the infrared absorption rate of the fitting region greater compared with the region other than the fitting region. Accordingly, it is possible to suppress positional deviations due to thermal expansion of jigs and parts, which serves to suppress manufacturing errors, so that the precision of positioning between the objective lens and the image-acquisition device can be improved.

In the above second aspect, conditional expression (6) below may be satisfied:

$$Rbo > 3 \times Rai \qquad (6)$$

where Rai signifies the maximum height of the surface roughness of the outer surface of the region other than the fitting region in the outer surface of the frame that is located on the inner side, and Rbo signifies the maximum height of the surface roughness of the outer surface of the frame that is located on the outer side. The maximum height here refers to a value ($\mu$m) indicating the difference between a maximum value and a minimum value of the surface roughness.

This makes it possible to increase the surface area of the outer surface of the fitting region, so that it is possible to make the infrared absorption rate of the fitting region greater compared with the region other than the fitting region.

In the above second aspect, conditional expression (7) below may be satisfied:

$$2.5 \times P \times Fno < 0.03 \qquad (7)$$

where P signifies the pitch of the image-acquisition device, and Fno signifies the effective F number of the objective optical system.

It is possible to improve the positioning precision also in an optical system that requires positioning with such high precision satisfying conditional expression (7) given above.

{Advantageous Effects of Invention}

According to the present invention, an advantage is afforded in that manufacturing errors are suppressed, so that the precision of positioning between an image-acquisition device and an objective lens is improved.

{Brief Description of Drawings}

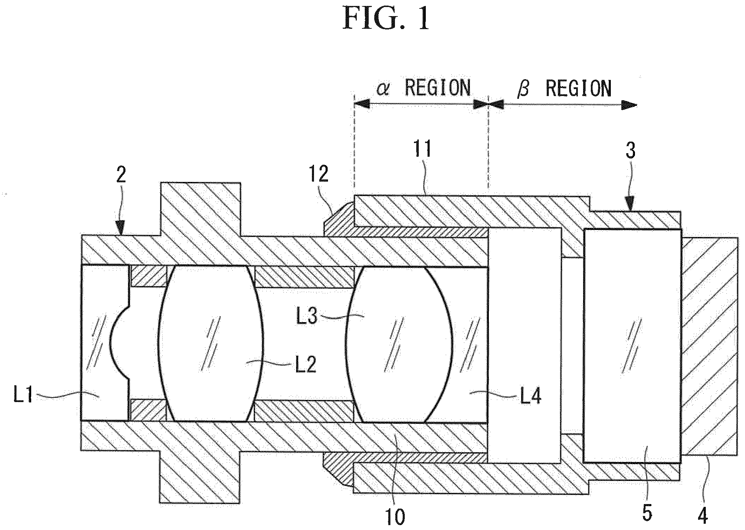

{Fig. 1} Fig. 1 is a sectional view showing the overall configuration of an endoscopic image-acquisition unit according to a first embodiment of the present invention.

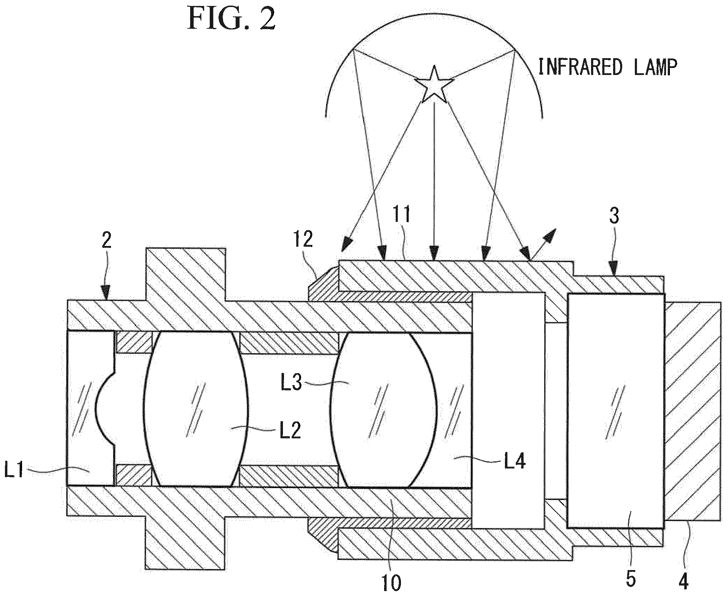

{Fig. 2} Fig. 2 is an illustration of a case where the endoscopic image-acquisition unit according to the first embodiment of the present invention is irradiated with infrared rays.

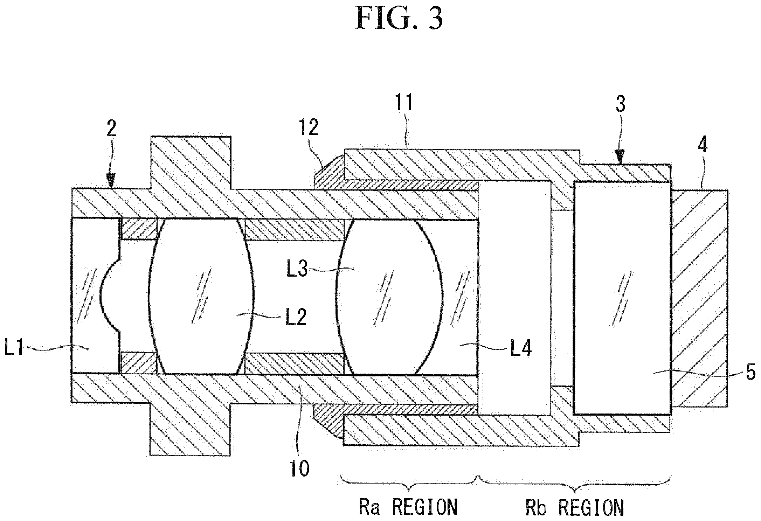

{Fig. 3} Fig. 3 is a sectional view showing the overall configuration of an endoscopic image-acquisition unit according to a second embodiment of the present invention.

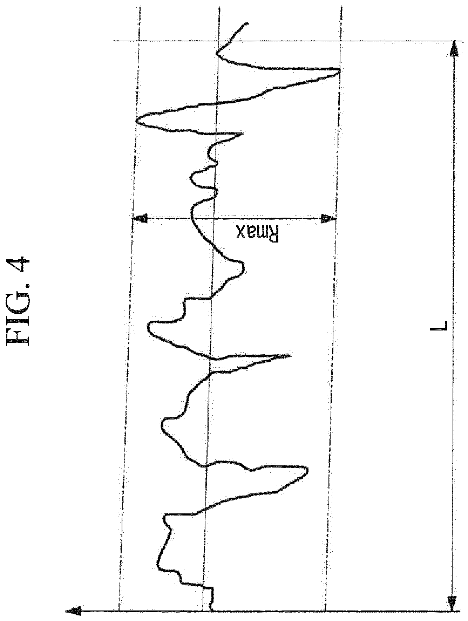

{Fig. 4} Fig. 4 is a graph for explaining a maximum value of surface roughness.

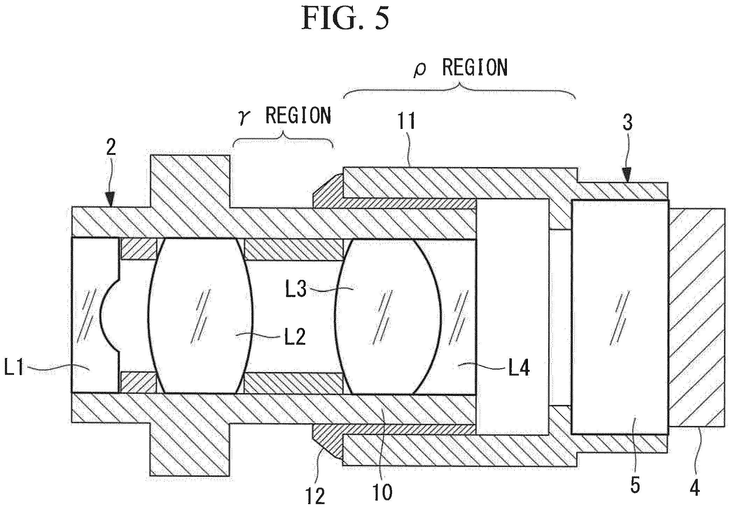

{Fig. 5} Fig. 5 is a sectional view showing the overall configuration of an endoscopic image-acquisition unit according to a third embodiment of the present invention.

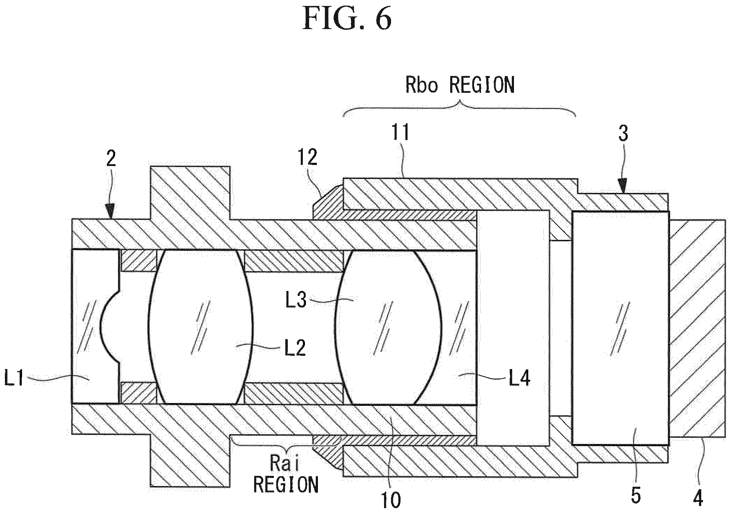

{Fig. 6} Fig. 6 is a sectional view showing the overall configuration of an endoscopic image-acquisition unit according to a fourth embodiment of the present invention.

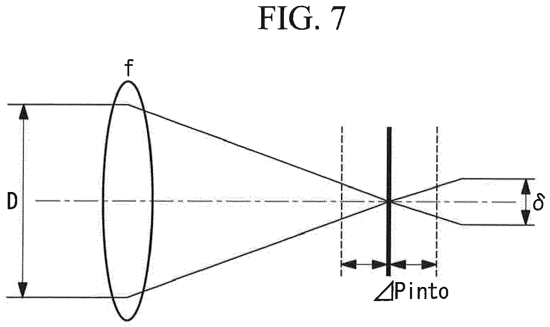

{Fig. 7} Fig. 7 is an illustration of an objective lens, the focal distance, and the tolerance for focal deviation in the endoscopic image-acquisition unit according to each of the embodiments of the present invention.

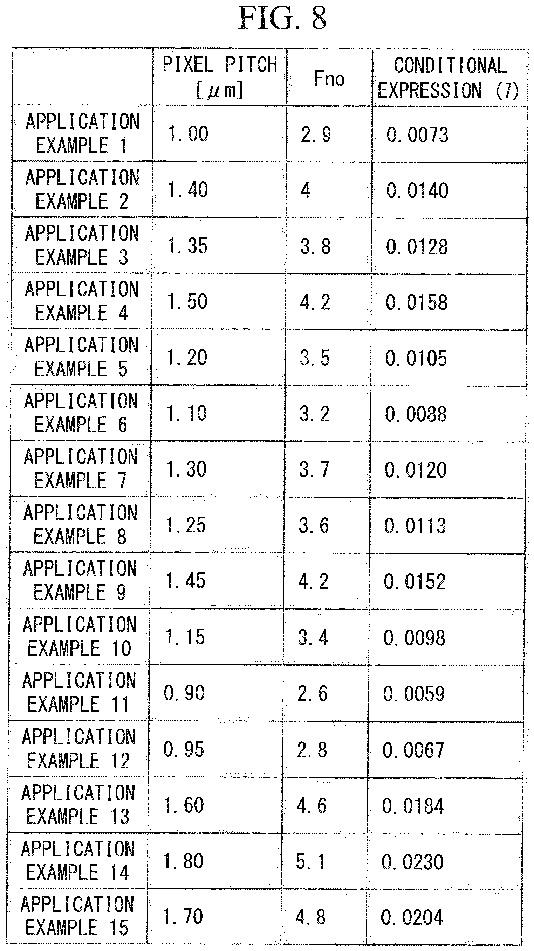

{Fig. 8} Fig. 8 is a graph showing a list of examples of an optical system that requires positioning with high precision satisfying conditional expression (7).

{Description of Embodiments}

(First Embodiment)

An endoscopic image-acquisition unit according to a first embodiment of the present invention will be described below with reference to the drawings.

As shown in Fig. 1, the endoscopic image-acquisition unit according to this embodiment includes an objective-lens-unit frame 2 that holds objective lenses and an image-acquisition-device holding frame 3 that is fitted with the objective-lens-unit frame 2 and that holds an image-acquisition device. This embodiment will be described assuming that the objective-lens-unit frame 2 is located on the inner side and the image-acquisition-device holding frame 3 is located on the outer side when these frames are fitted together.

The objective-lens-unit frame 2 holds multiple lenses L1, L2, L3, and L4. In the objective-lens-unit frame 2, an end region on the image-acquisition-surface side constitutes a fitting region 10 that is fitted with the image-acquisition-device holding frame 3.

The image-acquisition-device holding frame 3 holds an image-acquisition device 4 and a cover glass plate 5. In the image-acquisition-device holding frame 3, an end region on the object-surface side constitutes a fitting region 11 that is fitted with the objective-lens-unit frame 2.

Of the outer surface of the image-acquisition-device holding frame 3, which is the frame that is located on the outer side when the objective-lens-unit frame 2 and the image-acquisition-device holding frame 3 are fitted together, the outer surface of the fitting region 11 and the outer surface of the region other than the fitting region 11 are configured to satisfy the following conditional expression:

$$\alpha/\beta > 2 \qquad (1)$$

where $\alpha$ signifies the infrared absorption rate per unit area of the outer surface of the fitting region, and $\beta$ signifies the infrared absorption rate per unit area of the outer surface of the region other than the fitting region.

In this embodiment, the image-acquisition-device holding frame 3 is made of a metallic material. Of the outer surface of the image-acquisition-device holding frame 3, the outer surface of the fitting region 11 is plated with chromium, and the outer surface of the region other than the fitting region 11 is not plated or otherwise treated, so that the machined surface of the metallic material is exposed, constituting a lustrous metallic surface. That is, of the outer surface of the image-acquisition-device holding frame 3, compared with the metallic surface of the outer surface of the region other than the fitting region 11, the outer surface of the fitting region 11 absorbs infrared rays more efficiently since it is chromium-plated and is black. For the convenience of description, in Fig. 1, the outer surface of the fitting region is labeled as "$\alpha$ region," and the outer surface of the region other than the fitting region is labeled as "$\beta$ region."

For example, in the case where stainless steel is used as the metallic material of the image-acquisition-device holding frame 3, which is located on the other side, and the outer surface thereof is irradiated with infrared rays having a wavelength of about 1 to 2 $\mu$m, the infrared absorption rate per unit area of the outer surface (metallic surface) of the region other than the fitting region 11, which is not chromium-plated, is about 7%. On the other hand, the infrared absorption rate per unit area of the outer surface of the fitting region 11, where the stainless steel is chromium-plated, can be improved to about 16% by the effect of a chromium-oxide film that is used for chromium plating.

At this time, $\alpha$ = 16%, $\beta$ = 7%, and $\alpha/\beta$ = 2.3, so that conditional expression (1) given above is satisfied.

It is possible to further improve the infrared absorption rate per unit area of the outer surface of the fitting region 11 by applying conditional expression (2) instead of conditional expression (1) above:

$$\alpha/\beta > 4 \qquad (2)$$

The endoscopic image-acquisition unit configured as described above is assembled as follows. As shown in Fig. 2, an adhesive 12 made of a thermosetting resin is applied to the outer surface of the fitting region 10 or the inner surface of the fitting region 11, the spacing between the objective-lens-unit frame 2 and the image-acquisition-device holding frame 3 is adjusted, and then the adhesive 12 is cured by heating it from the outside by using infrared rays. Although only one infrared lamp is shown in Fig. 2 for simplicity, in practice, multiple infrared lamps are disposed circumferentially for heating since it is necessary to heat the entire fitting region.

As described above, in the image-acquisition-device holding frame 3, which is located on the outer side when fitted with the objective-lens-unit frame 2, the outer surface of the fitting region 11 is configured so that it is heated more easily by infrared irradiation compared with the region other than the fitting region 11. Therefore, it is possible to efficiently heat only the fitting region 11 by infrared irradiation of the fitting region 11. Accordingly, it is possible to suppress positional deviations due to thermal expansion of jigs and parts, which serves to suppress manufacturing errors, so that the accuracy of positioning between the objective lenses and the image-acquisition device can be improved.

In this embodiment, the image-acquisition-device holding frame 3, which is located on the outer side, is made of a metallic material, and the metallic material is plated with chromium. Alternatively, however, other kinds of blackening treatment may be employed, such as chromate treatment, alumite treatment, or nickel plating. It is possible to employ treatment that enables black plating in relation to a metallic material that is used for the frame that is located on the outer side among the image-acquisition-device holding frame 3 and the objective-lens-unit frame 2. Furthermore, although stainless steel is described as the metallic material, without limitation to stainless steel, other kinds of metallic material, such as copper, brass, aluminum, and iron, may be used.

Furthermore, although the objective-lens-unit frame 2 is located on the inner side and the image-acquisition-device holding frame 3 is located on the outer side when the objective-lens-unit frame 2 and the image-acquisition-device holding frame 3 are fitted together in this embodiment, there is no limitation to this configuration. Alternatively, the configuration may be such that the objective-lens-unit frame 2 is located on the outer side and the image-acquisition-device holding frame 3 is located on the inner side. In this case, the outer surface of the fitting region 10 of the objective-lens-unit frame 2, which is located on the outer side when fitted, is chromium-plated or otherwise treated so that the infrared absorption rate will be higher.

(Modification)

In the first embodiment described above, the outer surface of the fitting region 11 of the image-acquisition-device holding frame 3 is chromium-plated. Alternatively, instead of such plating, an infrared-absorbing material may be applied to the outer surface of the fitting region 11.

Specifically, an infrared-absorbing material is applied to the outer surface of the fitting region of the frame that is located on the outer side when the objective-lens-unit frame and the image-acquisition-device holding frame are fitted together such that conditional expression (1) given earlier is satisfied. With this configuration, the outer surface of the fitting region is heated more easily by infrared irradiation compared with the outer surface of the region other than the fitting region.

As the infrared-absorbing material, for example, a black paint, or a black paint in which a metal oxide, etc. is introduced so that infrared rays can be absorbed efficiently, may be used. In this case, black ink may be used alone, or powder of an oxide of a metal, such as chromium, copper, iron, nickel, or molybdenum, may be introduced to further improve the absorption rate, whereby the infrared absorption rate per unit area can be further improved. By applying such a paint only to the outer surface of the fitting region of the frame that is located on the outer side and forming a metallic surface on the outer surface of the region other than the fitting region, the infrared absorption rate of the fitting region can be made higher than that of the region other than the fitting region.

(Second Embodiment)

An endoscopic image-acquisition unit according to a second embodiment of the present invention will be described below with reference to the drawings. In the following description, parts that are configured the same as those of the endoscopic image-acquisition unit according to the first embodiment described above are designated by the same reference signs, and descriptions thereof will be omitted. Furthermore, this embodiment will also be described assuming that, similarly to the first embodiment, the objective-lens-unit frame 2 is located on the inner side and the image-acquisition-device holding frame 3 is located on the outer side when these frames are fitted together.

As shown in Fig. 3, the outer surface of the image-acquisition-device holding frame 3, located on the outer side when fitted, is configured such that the surface roughness of the outer surface of the fitting region 11 is greater than the surface roughness of the outer surface of the region other than the fitting region (the region labeled as "RA REGION" in the figure constitutes a rough surface). More specifically, the outer surface of the image-acquisition-device holding frame 3, which is the frame that is located on the outer side when the objective-lens-unit frame 2 and the image-acquisition-device holding frame 3 are fitted together, is configured such that the outer surface of the fitting region 11 and the outer surface of the region other than the fitting region 11 satisfy conditional expression (1) given earlier and also satisfy conditional expression (3) given below:

$$Ra > 3 \times Rb \qquad (3)$$

where Ra signifies the maximum height of the surface roughness of the outer surface of the fitting region 11, and Rb signifies the maximum height of the surface roughness of the outer surface of the region other than the fitting region 11.

The maximum height here refers to a value (μm) indicating the difference between a maximum value and a minimum value of surface roughness. As shown in Fig. 4, the maximum height represents the maximum value (Rmax in Fig. 4), in μm, of the difference between the maximum height and minimum height in the case where surface roughness is measured over a certain reference range L.

In Fig. 3, the outer surface of the fitting region 11, having the maximum height Ra of surface roughness, is labeled as "Ra REGION," and the outer surface of the region other than the fitting region 11, having the maximum height Rb of surface roughness, is labeled as "Rb REGION."

It is possible to make the surface roughness of the outer surface of the fitting region 11 greater than the surface roughness of the outer surface of the region other than the fitting region 11, for example, by varying the rotation rate or moving amount of a cutting blade between the outer surface of the fitting region 11 and the outer surface of the region other than the fitting region 11 when machining the frame or by performing sandblasting after machining. By subsequently performing blackening treatment, such as plating, it is possible to configure an image-acquisition-device holding frame in which the outer surface of the fitting region 11 has a higher infrared absorption rate than the outer surface of the region other than the fitting region 11.

In this embodiment, for example, stainless steel is used as the metallic material of the image-acquisition-device holding frame 3, which is located on the outer side. The infrared absorption rate per unit area of the outer surface of the fitting region 11, where the stainless steel is plated with chromium, is improved to about 16% due to the effect of a chromium oxide film used for chromium plating. Furthermore, when Ra = 25 μm and Rb = 6.3 μm, the infrared absorption rate per unit area of the Ra region is:

$$0.16 \times 1.16 \times 1.16 \times 1.16 \times 1.16 = 0.29$$

This indicates that, as opposed to the Rb region, which is not plated or otherwise treated, the infrared absorption rate per unit area is improved to about 29%. In this case, Ra = 3.97 × Rb, so that the condition Ra > 3 × Rb is satisfied. At this time, $\alpha$ = 29%, and the infrared absorption rate per unit area of the outer surface (metallic surface) of the region other than the fitting region 11, which is not plated, is $\beta$ = 7%, so that $\alpha/\beta$ = 4.14, which satisfies conditional expression (2), so that it is possible to heat the required region with this configuration.

This embodiment has been described in the context of an example in which machining is performed such that Ra = 25 μm and Rb = 6.3 μm. Alternatively, for example, by making Ra = 25 μm and Rb = 3.2 μm, the infrared absorption rate per unit area can be improved to about 45%. In this case, $\alpha/\beta$ = 6.4, so that conditional expression (2) is well satisfied, as well as conditional expression (1).

The endoscopic image-acquisition unit configured as described above is assembled by applying an adhesive 12 made of a thermosetting resin to the outer surface of the fitting region 10 or the inner surface of the fitting region 11, adjusting the spacing between the objective-lens-unit frame 2 and the image-acquisition-device holding frame 3, and then curing the adhesive 12 by heating it from the outside by using infrared rays.

As described above, the surface roughness of the outer surface of the fitting region 11 of the image-acquisition-device holding frame 3, which is located on the outer side when fitted with the objective-lens-unit frame 2, is greater than the surface roughness of the outer surface of the region other than the fitting region 11. Therefore, it is possible to efficiently heat only the fitting region 11 by infrared irradiation of the fitting region 11. Accordingly, it is possible to suppress positional deviations due to thermal expansion of jigs and parts, which serves to suppress manufacturing errors, so that the accuracy of positioning between the objective lenses and the image-acquisition device can be improved.

(Third Embodiment)

An endoscopic image-acquisition unit according to a third embodiment of the present invention will be described below with reference to the drawings. In the following description, parts that are configured the same as those of the endoscopic image-acquisition unit according to the first embodiment described above are designated by the same reference signs, and descriptions thereof will be omitted. Furthermore, this embodiment will also be described assuming that, similarly to the first embodiment, the objective-lens-unit frame 2 is located on the inner side and the image-acquisition-device holding frame 3 is located on the outer side when these frames are fitted together.

As shown in Fig. 5, in this configuration, the infrared absorption rate $\rho$ of the outer surface of the image-acquisition-device holding frame 3, which is located on the outer side when fitted, is greater than the infrared absorption rate $\gamma$ of the outer surface of the region other than the fitting region 11 of the outer surface of the objective-lens-unit frame 2, which is located on the inner side when fitted. In Fig. 5, the outer surface of the fitting region 11 of the image-acquisition-device holding frame 3, having the infrared absorption rate p, is labeled as "$\rho$ REGION," and the outer surface of the region other than the fitting region 10 of the objective-lens-unit frame 2, having the infrared absorption rate $\gamma$, is labeled as "$\gamma$ REGION."

That is, the outer surface of the frame that is located on the outer side when the objective-lens-unit frame 2 and the image-acquisition-device holding frame 3 are fitted together and the outer surface of the region other than the fitting region in the outer surface of the frame that is located on the inner side are configured to satisfy conditional expression (4) given below:

$$\rho/\gamma > 1.5 \qquad (4)$$

where $\rho$ signifies the infrared absorption rate per unit area of the outer surface of the frame that is located on the outer side, and $\gamma$ signifies the infrared absorption rate per unit area of the outer surface of the region other than the fitting region in the outer surface of the frame that is located on the inner side. Although $\gamma$ here signifies the infrared absorption rate per unit area of the outer surface of the region other than the fitting region in the outer surface of the frame that is located on the inner side when fitted, alternatively, the entire outer surface of the frame that is located on the inner side when fitted, including the fitting region thereof, may have the same infrared absorption rate $\gamma$. This indicates that either is acceptable since the outer surface of the fitting region of the frame that is located on the inner side when fitted is not irradiated with infrared rays in the case of infrared irradiation from the outside.

In this embodiment, the image-acquisition-device holding frame 3, which is located on the outer side, and the objective-lens-unit frame 2, which is located on the inner side, are made of a metallic material. In this embodiment, for example, the configuration may be such that stainless steel is used as a metallic material for both frames, the peripheral surface of the region other than the fitting region of the objective-lens-unit frame 2, which is located on the inner side, is not plated or otherwise treated, so that the machined surface of the metallic material is exposed, constituting a lustrous metallic surface, and the outer surface of the fitting region of the image-acquisition-device holding frame 3, which is located on the outer side, is plated with chromium. In this case, as described above, the peripheral surface of the fitting region of the objective-lens-unit frame 2, which is located on the inner side, is not plated or otherwise treated, so that the machined surface of the metallic material is exposed, constituting a lustrous metallic surface, similarly to the peripheral surface of the region other than the fitting region.

In assembling the endoscopic image-acquisition device, it is difficult to irradiate only the fitting region with infrared rays, and there are cases where the peripheral surface of the region other than the fitting region of the frame that is located on the inner side is also irradiated with infrared rays. Thus, it is desired to suppress, as much as possible, absorption of infrared rays by the outer surface of the region other than the fitting region of the frame that is fitted on the inner side so that infrared rays will be absorbed efficiently only by the outer surface of the frame that is located on the outer side.

In this embodiment, the outer surface of the region other than the fitting region 10 of the objective-lens-unit frame 2, which is located on the inner side, is simply the metallic surface, so that it is possible to achieve the infrared absorption rate per unit area of $\gamma$ = 7%. Furthermore, the outer surface of the image-acquisition-device holding frame 3, which is located on the outer side, is plated with chromium, so that it is possible to achieve the infrared absorption rate per unit area of $\rho$ = 16%.

Thus, $\rho/\gamma$ = 2.28, so that conditional expression (4) given earlier is well satisfied. In this case, even if the region other than the fitting region is irradiated with infrared rays, it is possible to heat the fitting region more efficiently compared with the other region.

Furthermore, by applying conditional expression (5) below instead of conditional expression (4) given earlier, it is possible to further increase the infrared absorption rate in the fitting region.

$$\rho/\gamma > 2 \quad (5)$$

Also in this embodiment, the image-acquisition-device holding frame 3, which is located on the outer side, is made of a metallic material, and the metallic material is plated with chromium. Alternatively, however, other kinds of blackening treatment may be employed, such as chromate treatment, alumite treatment, or nickel plating. Furthermore, although an example in which stainless steel is used for the image-acquisition-device holding frame 3 has been described, without limitation to this example, other kinds of metallic material, such as copper, brass, aluminum, and iron, may be used.

Furthermore, although this embodiment has been described in the context of an example in which the infrared absorption rate per unit area is increased by chromium-plating the outer surface of the image-acquisition-device holding frame 3, which is located on the outer side, other configurations may be employed as long as it is possible to increase the infrared absorption rate per unit area, such as a configuration in which an infrared absorbing material is applied. Examples of such an infrared absorbing material have been given earlier.

(Fourth Embodiment)

An endoscopic image-acquisition unit according to a fourth embodiment of the present invention will be described below with reference to the drawings. In the following description, parts that are configured the same as those of the endoscopic image-acquisition unit according to the first embodiment described above are designated by the same reference signs, and descriptions thereof will be omitted. Furthermore, this embodiment will also be described assuming that, similarly to the first embodiment, the objective-lens-unit frame 2 is located on the inner side and the image-acquisition-device holding frame 3 is located on the outer side when these frames are fitted together.

As shown in Fig. 6, in this configuration, the surface roughness of the outer surface of the image-acquisition-device holding frame 3, which is located on the outer side when fitted, is greater than the surface roughness of the outer surface of the region other than the fitting region 10 of the objective-lens-unit frame 2, which is located on the inner side. Specifically, the outer surface of the image-acquisition-device holding frame 3, which is the frame that is located on the outer side when the objective-lens-unit frame 2 and the image-acquisition-device holding frame 3 are fitted together, and the outer surface of the region other than the fitting region 10 of the objective-lens-unit frame 2, which is the frame that is located on the inner side, are configured to satisfy conditional expression (4) given earlier and also satisfy conditional expression (6) given below:

$$Rbo > 3 \times Rai \quad (6)$$

where Rai signifies the maximum height of the surface roughness of the outer surface of the region other than the fitting region in the outer surface of the frame that is located on the inner side, and Rbo signifies the maximum height of the surface roughness of the outer surface of the frame that is located on the outer side. The maximum height here refers to a value (μm) indicating the difference between a maximum value and a minimum value of the height of surface roughness. Although Rai here signifies the maximum height of the surface roughness of the outer surface of the region other than the fitting region 10 in the outer surface of the frame that is located on the inner side, alternatively, the entire outer surface of the frame that is located on the inner side, including the fitting region thereof, may have the same surface roughness Rai. This indicates that either is acceptable since the outer surface of the fitting region of the frame that is located on the inner side when fitted is not irradiated with infrared rays in the case of infrared irradiation from the outside.

In Fig. 6, the outer surface of the region other than the fitting region 10 of the objective-lens-unit frame 2, having the maximum height Rai of the surface roughness, is labeled as "Rai REGION," and the outer surface of the image-acquisition-device holding frame 3, having the maximum height Rbo of the surface roughness, is labeled as "Rbo REGION."

For example, in the case where the maximum height of the surface roughness of the outer surface of the image-acquisition-device holding frame 3, which is located on the outer side, is Rbo = 25 μm, and the maximum height of the surface roughness of the outer surface of the region other than the fitting region 10 in the outer surface of the objective-lens-unit frame 2, which is located on the inner side, is Rai = 6.3 μm, conditional expression (6) given earlier is satisfied.

In this embodiment, the objective-lens-unit frame 2 and the image-acquisition-device holding frame 3 are both made of a stainless steel material, and black plating is applied at least to the outer surfaces thereof. In this case, the ratio of the infrared absorption rate per unit area of the outer surface of the objective-lens-unit frame 2, which is located on the inner side at the fitting region, and the infrared absorption rate per unit area of the outer surface of the image-acquisition-device holding frame 3, which is located on the outer side, is $\rho/\gamma = 1.81$.

As another example, in the case where the maximum height of the surface roughness of the outer surface of the image-acquisition-device holding frame 3, which is located on the outer side, is Rbo = 25 μm, and the maximum height of the surface roughness of the outer surface of the region other than the fitting region 10 in the outer surface of the objective-lens-unit frame 2, which is located on the inner side, is Rai = 3.2 μm, conditional expression (6) given earlier is satisfied, and $\rho/\gamma$ in this case is 2.4.

Also with this configuration, in which blackening treatment is applied to both the objective-lens-unit frame 2 and the image-acquisition-device holding frame 3, it is possible to efficiently heat the fitting region, i.e., only the region that is to be bonded and fixed, thereby curing the adhesive 12.

Furthermore, in this embodiment, the surface roughness of the peripheral surface of the fitting region of the objective-lens-unit frame 2, which is located on the inner side in the fitting region, may be either the same as or different from the surface roughness Rai of the peripheral surface of the region other than the fitting region.

As described earlier, for black plating of the metal, it is possible to choose an appropriate treatment that enables blackening with a metallic material, such as chromium plating, chromate treatment, alumite treatment, or nickel plating. Furthermore, for the objective-lens-unit frame 2 and the image-acquisition-device holding frame 3, it is possible to use a variety of metallic materials, such as copper, brass, aluminum, and iron, without limitation to stainless steel.

By configuring each of the endoscopic image-acquisition units according to the embodiments described above such that conditional expression (7) given below is satisfied, it is possible to improve the precision of positioning in an optical system that requires positioning with high precision.

$$2.5 \times P \times Fno < 0.03 \qquad (7)$$

where P signifies the pitch of the image-acquisition device, and Fno signifies the effective F number of the objective optical system.

This is because, as shown in Fig. 7, when the tolerance for the focal deviation of the endoscopic image-acquisition unit is signified by $\Delta_{pinto}$, the following equations hold:

$$\delta/D = \Delta_{pinto}/f$$

$$\Delta_{pinto}/f = \delta \cdot f/D$$

and assuming $\delta = 2.5P$, the following equation is obtained:

$$\Delta_{pinto} = Fno \times 2.5 \times P$$

where D signifies the effective aperture of the objective lenses, f signifies the focal distance, and $\delta$ signifies the diameter of blurring that is tolerable on the image plane.

The reason for $\delta = 2.5P$ is as follows. In the case where an image of a black and white chart is formed on the image-acquisition device by the objective optical system, for each pixel of the image-acquisition device, the reference quantity of blurring is $\delta = 2P$. Image-acquisition devices that use luminance signals are a type of image-acquisition device that uses such a reference quantity. Furthermore, in the case of an image-acquisition device having color filters at each pixel thereof, it is necessary to generate luminance signals from the color filters, and the reference quantity for blurring is generally considered to be at the level of $\delta = 3P$. Accordingly, the intermediate value, 5 = 2.5P, is used as a reference quantity of blurring that is compatible with all image-acquisition devices.

Fig. 8 shows a list of application examples 1 to 15 of an optical system that requires positioning with such high precision that conditional expression (7) is satisfied. It is possible to improve the positioning precision by applying the above-described embodiments of the present invention to these application examples.

{Reference Signs List}

2 Objective-lens-unit frame
3 Image-acquisition-device holding frame
4 Image-acquisition device
5 Cover glass plate
10 Fitting region

11 Fitting region
12 Adhesive
L1 Lens
L2 Lens
L3 Lens
L4 Lens

## Claims

**1.** An endoscopic image-acquisition unit comprising:

an objective-lens-unit frame that holds an objective lens; and
an image-acquisition-device holding frame that is fitted with the objective-lens-unit frame and that holds an image-acquisition device,
wherein the objective-lens-unit frame and the image-acquisition-device holding frame are bonded and fixed with a thermosetting resin that is applied to a fitting region of these frames, and
wherein, of the outer surface of the frame that is located on the outer side when the objective-lens-unit frame and the image-acquisition-device holding frame are fitted together, the outer surface of the fitting region and the outer surface of the region other than the fitting region satisfy the following conditional expression:

$$\alpha/\beta > 2 \quad (1)$$

where $\alpha$ signifies the infrared absorption rate per unit area of the outer surface of the fitting region, and $\beta$ signifies the infrared absorption rate per unit area of the outer surface of the region other than the fitting region.

**2.** An endoscopic image-acquisition unit according to Claim 1, satisfying conditional expression (2) below:

$$\alpha/\beta > 4 \quad (2)$$

**3.** An endoscopic image-acquisition unit according to Claim 1 or 2, satisfying conditional expression (3) below:

$$Ra > 3 \times Rb \quad (3)$$

where Ra signifies the maximum height of the surface roughness of the outer surface of the fitting region in the outer surface of the frame that is located on the outer side, Rb signifies the maximum height of the surface roughness of the outer surface of the region other than the fitting region in the outer surface of the frame that is located on the outer side, and the maximum height here refers to a value ($\mu$m) indicating the difference between a maximum value and a minimum value of the height of the surface roughness.

**4.** An endoscopic image-acquisition unit comprising:

an objective-lens-unit frame that holds an objective lens; and
an image-acquisition-device holding frame that is fitted with the objective-lens-unit frame and that holds an image-acquisition device,
wherein the objective-lens-unit frame and the image-acquisition-device holding frame are bonded and fixed with a thermosetting resin that is applied to a fitting region of these frames, and
wherein the outer surface of the frame that is located on the outer side when the objective-lens-unit frame and the image-acquisition-device holding frame are fitted together and the outer surface of the region other than the fitting region in the outer surface of the frame that is located on the inner side satisfy conditional expression (4) below:

$$\rho/\gamma > 1.5 \quad (4)$$

where $\rho$ signifies the infrared absorption rate per unit area of the outer surface of the frame that is located on the outer side, and $\gamma$ signifies the infrared absorption rate per unit area of the outer surface of the region other than the fitting region in the outer surface of the frame that is located on the inner side.

**5.** An endoscopic image-acquisition unit according to Claim 4, satisfying conditional expression (5) below:

$$\rho/\gamma > 2 \qquad (5)$$

**6.** An endoscopic image-acquisition unit according to Claim 4 or 5, satisfying conditional expression (6) below:

$$Rbo > 3 \times Rai \qquad (6)$$

where Rai signifies the maximum height of the surface roughness of the outer surface of the region other than the fitting region in the outer surface of the frame that is located on the inner side, Rbo signifies the maximum height of the surface roughness of the outer surface of the frame that is located on the outer side, and the maximum height here refers to a value ($\mu$m) indicating the difference between a maximum value and a minimum value of the height of the surface roughness.

**7.** An endoscopic image-acquisition unit according to any one of Claims 1 to 6, satisfying conditional expression (7) below:

$$2.5 \times P \times Fno < 0.03 \qquad (7)$$

where P signifies the pitch of the image-acquisition device, and Fno signifies the effective F number of the objective lens.

FIG. 1

α REGION
β REGION
2
3
4
5
10
11
12
L1
L2
L3
L4

INFRARED LAMP
3
4
5
L4
11
10
12
L3
L2
2
L1

FIG. 2

5
4
3
11
12
2
L1
L2
L3
L4
10
Rb REGION
Ra REGION

FIG. 3

Rmax
L

FIG. 4

ρ REGION
γ REGION
2
3
4
5
10
11
12
L1
L2
L3
L4

FIG. 5

4
5
3
L4
Rbo REGION
11
10
12
L3
L2
Rai REGION
2
L1

FIG. 6

FIG. 7

f
D
δ
⊿Pinto

# FIG. 8

| | PIXEL PITCH [μm] | Fno | CONDITIONAL EXPRESSION (7) |
|---|---|---|---|
| APPLICATION EXAMPLE 1 | 1.00 | 2.9 | 0.0073 |
| APPLICATION EXAMPLE 2 | 1.40 | 4 | 0.0140 |
| APPLICATION EXAMPLE 3 | 1.35 | 3.8 | 0.0128 |
| APPLICATION EXAMPLE 4 | 1.50 | 4.2 | 0.0158 |
| APPLICATION EXAMPLE 5 | 1.20 | 3.5 | 0.0105 |
| APPLICATION EXAMPLE 6 | 1.10 | 3.2 | 0.0088 |
| APPLICATION EXAMPLE 7 | 1.30 | 3.7 | 0.0120 |
| APPLICATION EXAMPLE 8 | 1.25 | 3.6 | 0.0113 |
| APPLICATION EXAMPLE 9 | 1.45 | 4.2 | 0.0152 |
| APPLICATION EXAMPLE 10 | 1.15 | 3.4 | 0.0098 |
| APPLICATION EXAMPLE 11 | 0.90 | 2.6 | 0.0059 |
| APPLICATION EXAMPLE 12 | 0.95 | 2.8 | 0.0067 |
| APPLICATION EXAMPLE 13 | 1.60 | 4.6 | 0.0184 |
| APPLICATION EXAMPLE 14 | 1.80 | 5.1 | 0.0230 |
| APPLICATION EXAMPLE 15 | 1.70 | 4.8 | 0.0204 |

**INTERNATIONAL SEARCH REPORT**

International application No. PCT/JP2015/052962

A. CLASSIFICATION OF SUBJECT MATTER
*A61B1/00*(2006.01)i, *G02B23/24*(2006.01)i

According to International Patent Classification (IPC) or to both national classification and IPC

B. FIELDS SEARCHED

Minimum documentation searched (classification system followed by classification symbols)
A61B1/00, G02B23/24

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 9-192093 A (Olympus Optical Co., Ltd.), 29 July 1997 (29.07.1997), paragraph [0015]; fig. 1 (Family: none) | 1-7 |
| A | JP 2008-125902 A (Pentax Corp.), 05 June 2008 (05.06.2008), paragraphs [0019], [0031] (Family: none) | 1-7 |
| A | WO 2011/092904 A1 (Olympus Medical Systems Corp.), 04 August 2011 (04.08.2011), paragraphs [0029] to [0031]; fig. 6 & JP 5189209 B2 | 1-7 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April 2015 (13.04.15) | 21 April 2015 (21.04.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI9192093 B